(19) Europäisches Patentamt
European Patent Office
Office européen des brevets

(11) Publication number: **0 272 921 B1**

(12) **EUROPEAN PATENT SPECIFICATION**

(45) Date of publication of patent specification: **24.04.91**

(51) Int. Cl.5: **C07D 213/80**, C07D 307/68, C07D 333/40, C07D 213/30, C07D 213/48, C07D 213/51, A61K 31/455, A61K 31/44, A61K 31/34, A61K 31/38

(21) Application number: **87311318.7**

(22) Date of filing: **22.12.87**

# (54) Ethynylheteroaromatic acids having retinoic acid-like activity.

(30) Priority: **24.12.86 US 946749**

(43) Date of publication of application:
**29.06.88 Bulletin 88/26**

(45) Publication of the grant of the patent:
**24.04.91 Bulletin 91/17**

(84) Designated Contracting States:
**AT BE CH DE ES FR GB GR IT LI LU NL SE**

(56) References cited:
**EP-A- 0 176 034**
**DE-A- 2 065 014**
**GB-A- 2 130 580**

**CHEMICAL ABSTRACTS, vol. 103, no. 3, 22 July 1985, Columbus, Ohio, USA; M.H.SILVEIRA et al.: "An improved synthesis of seven-membered ring-fused 12-s-cis conformationally locked 11-cis-retinoids", page 602, column 1, abstract no. 22805p**

(73) Proprietor: **ALLERGAN, INC**
**2525 Dupont Drive**
**Irvine, California 92715-1599(US)**

(72) Inventor: **Chandraratna, Roshantha A. S.**
**23015 Ashwood**
**El Toro, CA 92630(US)**

(74) Representative: **Hutchins, Michael Richard et al**
**Graham Watt & Co. London Road Riverhead**
**Sevenoaks, Kent TN13 2BN(GB)**

**EP 0 272 921 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background

This invention relates to novel compounds having retinoid-like activity. More specifically, the invention relates to compounds wherein three olefinic units from the acid-containing end unit in retinoic acid are replaced by an ethynylheteroaromatic-containing functionality. Such modifications to the retinoic acid structure have retinoid acid-like activity.

### Related Art

Nematocidal compounds disclosed in Japanese patent 56-123903, have the structure 2-(2-((1,1-dimethyl)-dimethylsilyl)oxy)ethyl-alpha-(4-(2,6,6-trimethyl-1-cyclohexene-1-yl)-3-buten-1-ynyl)-1-cyclopentene-1-methanol. This compound employs the 1-(2',6',6'-trimethyl-cyclohex-1'-enyl)-but-1-ene-3-yne moiety of the compounds disclosed herein. That fragment, however, is the only similarity between the Japanese disclosure and those recited herein. Such compounds are not dispositive of the instant invention.

### Summary of the Invention

This invention comprises compounds of formula I

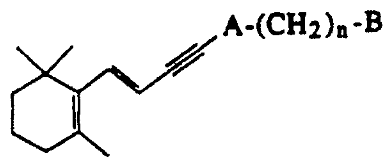

I

wherein A is pyridyl, furyl, thienyl, pyridazinyl, pyrimidinyl or pyrazinyl; n is 0-5; and B is H, -COOH and its esters, amides and pharmaceutically acceptable salts, -CHO and its acetal derivatives, $-COR_1$ and its ketal derivatives where $R_1$ is $-(CH_2)_nCH_3$ where n is defined above, or $-CH_2OH$ and acyl ester derivatives; or pharmaceutically acceptable salts thereof; the esters of -COOH and $-CH_2OH$ being derived from the saturated aliphatic alcohols or acids of ten or fewer carbon atoms or the cyclic or saturated aliphatic cyclic alcohols and acids of 5 to 10 carbon atoms, or being phenyl or $C_1$-6 alkylphenyl esters; the amides of -COOH being the unsubstituted amides, or the mono- and di-substituted amides derived from the saturated aliphatic radicals of ten or fewer carbon atoms or the cyclic or saturated aliphatic-cyclic radicals of 5 to 10 carbon atoms, or phenyl or $C_{1-6}$ alkylphenyl radicals; the acetals of - CHO and the ketals of $COR_1$ containing a group -CK in place of the carbonyl group, -CK being a group $(OR)_2$ wherein R is $C_{1-6}$ alkyl, or a group $-OR_{1a}O-$ wherein $R_{1a}$ is $C_{2-5}$ alkylene, straight chain or branched.

In a second aspect, this invention relates to the use of the compounds of formula I for treating dermatoses, such as acne, Darier's disease, psoriasis, icthyosis, eczema, atopic dermatitis and epithelial cancers. These compounds are also useful in the treatment of arthritic diseases and other immunological disorders (eg. lupus erythematosus), in promoting wound healing and in treating the dry eye syndrome.

This invention also relates to a pharmaceutical formulation comprising a compound of formula I in admixture with a pharmaceutically acceptable excipient.

In another aspect, this invention relates to the process for making a compound of formula I which process comprises reacting a compound of formula II with a compound of formula III in the presence of $Pd(PQ_3)_4$ (Q is phenyl) or a similar complex

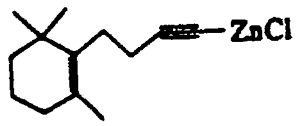

$X-A-(CH_2)_n-B$

II III

where x is a halogen, preferably I; A is pyridyl, furyl, thienyl, pyridazinyl, pyrimidinyl or pyrazinyl; n is the same as defined above; and B is H, or a protected acid, alcohol, aldehyde or ketone, to give a compound corresponding to formula I; or
homologating a compound of the formula

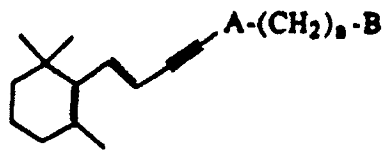

where n is 0-4 and A is a heterocycle as defined above; or
converting an acid of formula I to an acid or acid salt; or
converting an acid of formula I to an ester; or
converting an acid of formula I to an amide; or
reducing an acid to an alcohol or aldehyde; or
converting an alcohol to an ester; or
oxidizing an alcohol to an aldehyde; or
converting an aldehyde to an acetal; or
converting a ketone to a ketal.

One sub-group of compounds of the present invention is that group of compounds wherein A is pyridyl. Particular pyridyl compounds are those wherein B is -COOH, its $C_{1-6}$ alkyl esters and pharmaceutically acceptable salts, -CHO and $CH_2OH$.

A further sub-group of compounds of the present invention is that group of compounds wherein A is furyl. Particular furyl compounds are those wherein B is -COOH and its $C_{1-6}$ alkyl esters and pharmaceutically acceptable salts, -CHO or $-CH_2OH$.

Another sub-group of compounds of the present invention is that group of compounds wherein A is thienyl. Particular thienyl compounds are those wherein B is -COOH and its $C_{1-6}$ alkyl esters and pharmaceutically acceptable salts, -CHO or $CH_2OH$.

A still further sub-group of compounds of the present invention is that group wherein A is pyridazinyl, pyrimidinyl or pyrazinyl.

Preferred aliphatic esters are those derived from lower alkyl acids and alcohols. Here, and wherever else used, lower alkyl means having 1-6 carbon atoms. Also preferred are the phenyl or lower alkylphenyl esters.

Preferred amides are unsubstituted amides and those derived from substituted and unsubstituted lower alkyl amines.

A pharmaceutically acceptable salt may be prepared for any compounds in this invention having a functionality capable of forming such a salt, for example an acid or an amine functionality. A pharmaceutically acceptable salt is any salt which retains the activity of the parent compound and does not impart any deleterious or untoward effect on the subject to which it is administered and in the context in which it is administered.

Pharmaceutically acceptable salts may be derived from organic or inorganic acids or bases. The salt may be a mono- or polyvalent ion. Of particular interest are the inorganic ions, sodium, potassium, calcium, and magnesium. Organic salts may be made with amines, particularly ammonium salts such as mono-, diand trialkyl amines or ethanol amines. Salts may also be formed with caffeine, tromethamine and similar molecules.

The preferred compounds of this invention are those where the ethynyl group and the B group are attached to the 2 and 5 positions respectively of a pyridine ring (the 6 and 3 positions in the nicotinic acid

nomenclature being equivalent to the 2/5 designation in the pyridine nomenclature) or the 5 and 2 positions respectively of a thiophene group respectively; n is 0, 1 or 2; and B is -COOH, an alkali metal salt or organic amine salt, or a lower alkyl ester, or $-CH_2OH$ and the lower alkyl esters thereof. The more preferred compounds are:

ethyl 6-[4-(2,6,6-trimethylcyclohex-1-enyl)-but-3-en-1-ynyl]nicotinoate;
6-[4-(2, 6, 6-trimethylcyclohex-1-enyl)-but-3-en-1-ynyl]nicotinic acid;
ethyl 5-[4-(2,6,6-trimethylcyclohex-1-enyl)-but-3-en-1-ynyl]thiophene-2-carboxylate; and
5-[4-(2,6,6-trimethylcyclohex -1-enyl)-but-3-en-1-ynyl]thiophene-2-carboxylic acid.

The compounds of this invention may be administered systemically or topically, depending on such considerations as the condition to be treated, need for site-specific treatment, quantity of drug to be administered, and numerous other considerations.

In the treatment of dermatoses, it will generally be preferred to administer the drug topically, though in certain cases such as treatment of severe cystic acne, oral administration may also be used.

Any common topical formulation such as a solution, suspension, gel, ointment, or salve and the like may be used for topical treatment. Preparations of such topical formulations are well known and fully described in the art of pharmaceutical formulations as exemplified by, for example, Remington's Pharmaceutical Science, Edition 17, Mack Publishing Company, Easton, Pennsylvania. For topical application, these compounds could also be administered as a powder or spray, particularly in aerosol form.

If the drug is to be administered systemically, it may be confected as a powder, pill, tablet or the like, or as a syrup or elixir for oral administration. For intravenous or intraperitoneal administration, the compound will be prepared as a solution or suspension capable of being administered by injection. In certain cases, it may be useful to formulate these compounds in suppository form or as a sustained release formulation for deposit under the skin or intramuscular injection.

Other medicaments can be added to topical formulations for such secondary purposes as treating skin dryness, providing protection against light; other medications for treating dermatoses, preventing infection, reducing irritation, inflammation and the like.

Treatment of dermatoses or any other indications known or discovered to be susceptible to treatment by retinoic acid-like compounds will be effected by administration of the therapeutically effective dose of one or more compounds of the instant invention. A therapeutic concentration will be that concentration which effects reduction of the particular condition, or retards its expansion. In certain instances, the drug potentially could be used in a prophylactic manner to prevent onset of a particular condition. A given therapeutic concentration will vary from condition to condition and in certain instances may vary with the severity of the condition being treated and the patients susceptibility to treatment. Accordingly, a given therapeutic concentration will be best determined at the time and place through routine experimentation.

It is anticipated that, in the treatment of, for example, acne, or other such dermatoses, a formulation containing between 0.01 and 1.0 milligrams per milliter of formulation will constitute a therapeutically effective concentration. If administered systemically, an amount between 0.01 and 5 mg per kg per day of body weight would be expected to effect a therapeutic result.

The retinoic acid-like activity of these compounds was confirmed through the classic measure of retinoic acid activity involving the effects of retinoic acid on ornithine decarboxylase. The original work on the correlation between retinoic acid and decrease in cell proliferation was done by Verma & Boutwell, Cancer Research , 1977 , 37 , 2196-2201. That reference discloses that ornithine decarboxylase (ODC) activity increased precedent to polyamine biosynthesis. It has been established elsewhere that increases in polyamine synthesis can be correlated or associated with cellular proliferation. Thus, if ODC activity could be inhibited, cell hyperproliferation could be modulated. Although the causes for ODC activity increase are unknown, it is known that 12-0-tetradecanoylphorbol-13-acetate (TPA) induces ODC activity. Retinoic acid inhibits this induction of ODC activity by TPA. The compounds of this invention also inhibit TPA induction of ODC as demonstrated by an assay essentially following the procedure set out in Cancer Res .: 1662-1670, 1975.

## Specific Embodiments

It is anticipated that the compounds of this invention can be made by a number of different synthetic chemical pathways. To illustrate this invention, a series of steps which have been used to obtain certain representative compounds of formula I are outlined in scheme I. The synthetic chemist will readily appreciate that the conditions set out in this writing are specific embodiments which can be generalized to any and all of the compounds represented by formula I.

Reaction Scheme I outlines the general procedure for making the compounds of formula I.

Scheme I

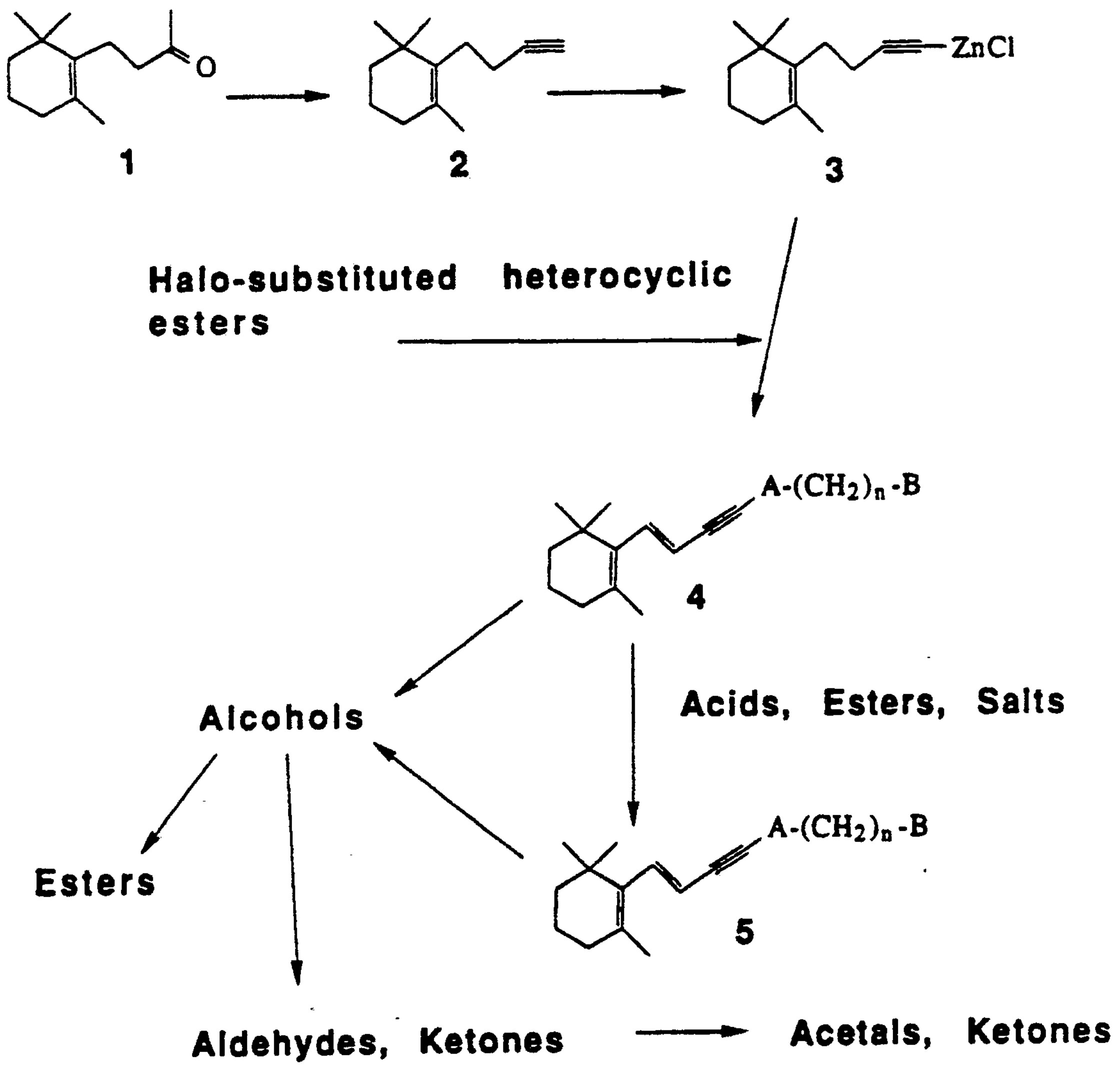


In this scheme, the halo on the "Halo-substituted heterocyclic esters" may be Br, Cl or I, but Br and I are preferred. A and B and n have the same meaning as recited on page 2 under the definition of the substituents of Formula I.

Stated generally, the acetylenic function is introduced by treating the ketone of formula 1 with a strong base and a dialkyl chlorophosphate followed by base treatment again. Then, by converting the acetylenic group to a heavy metal salt, the ZnCl salt, addition of the acetylenic function to an aromatic ring can be effected. Because of the basic nature of this ZnCl salt, the acidic properties of the B group must be minimized. Derivatizing acids, alcohols, aldehydes and ketone is necessary for optimizing reaction yields. Where it is necessary to extent the alkyl chain ($-CH_2)_n-$) after the preceeding step, that can be accomplished by an homologation reaction such as the Arndt-Eistert reaction.

The compound of formula 1 is sold by Aldrich Chemical Company under the name Beta-Ionone. The ketone is converted to a triple bond at reduced temperature under an inert atmosphere by means of lithium diisopropylamide (LDA) or a similar base. The reaction is carried out in an ether-type solvent such as a dialkyl ether or a cyclic ether, for example, tetrahydrofuran, pyran or the like.

More specifically, lithium diisopropylamide is generated in situ by mixing diisopropylamine in a dry solvent such as tetrahydrofuran, which is then cooled, to between -70 and -50°C under an inert atmosphere. An equimolar amount of an alkylithium compound such as n-butyl lithium in an appropriate solvent is then added at the reduced temperature and mixed for an appropriate time to permit formation of lithium diisopropylamide (LDA). The ketone of formula 1 (at least a 10% molar excess) is dissolved in the

reaction solvent, the solution cooled to that of the LDA mixture, and added to that solution. After brief mixing, the solution is then treated with a dialkyl chlorophosphate, preferably diethyl chlorophosphate in about a 20% molar excess. The reaction solution is then gradually brought to room temperature. This solution is then added to a second lithium diisopropylamide solution which is prepared in situ using dry solvent and under an inert atmosphere, preferrably argon, at reduced temperature (-78° C). Thereafter, the reaction mixture is again warmed to room temperature where it is stirred for an extended period of time, preferably between 10 and 20 hours, most preferably about 15 hours. The solution is then acidified and the product recovered by conventional separatory means.

The formula 3 compound is prepared under conditions which exclude all water and oxygen. A dry, ether-type solvent such as a dialkyl ether or a cyclic ether such as a furan or pyran, particularly tetrahydrofuran, may be used as the solvent. A solution of a compound of the formula 2 is first prepared under an inert atmosphere such as argon or nitrogen, and then a strong base such as n-butyl lithium is added (in about a 10% molar excess). This reaction is begun at a reduced temperature of between -10° and +10° C, preferably about 0° C. The reaction mixture is stirred for a short period, between 30 minutes and 2 hours, and then treated with about a 10% molar excess of fused zinc chloride dissolved in the reaction solvent. This mixture is stirred for an additional 1-3 hours at about the starting temperature, then the temperature is increased to about ambient temperature for 10-40 minutes.

The halo-substituted heterocyclic esters are prepared from their corresponding acids, the halogen being Cl, Br or I. These pyridyl, furyl, thienyl and diazinyl acids are all available from chemical manufacturers or can be prepared by published methods. Esterification is effected by refluxing the acid in a solution of the appropriate alcohol in the presence of thionyl chloride or by reacting the acid and alcohol in the presence of dicyclohexylcarbodiimide and dimethylaminopyridine. The ester is recovered and purified by conventional means. Other conventional methods can also be used to effect esterification.

To effect formation of formula 4, the alkyl halofuranoate, or a corresponding alkyl halo ester of thiophene, pyridine or the several diazine isomers, is dissolved in a dry solvent. The ester is used in an amount approximating the molar quantity of the starting quantity of compound 3. This solution is introduced into a suspension of tetrakis(triphenylphosphine)palladium (about a 5 to 10% molar amount relative to the reactants) in the reaction solvent at a temperature of between about -10° and +10° C. This mixture is stirred briefly, for about 15 minutes. To this just-prepared mixture is then added the pre-prepared solution of the compound 3, the zinc chloride salt, the addition being made at about room temperature. This solution is stirred for an extended period, between about 15 and 25 hours, at room temperature. The reaction is then quenched with acid and the product separated and purified by conventional means to give the compounds of formula 4.

Taking the esters of formula 4, saponifying them, and taking the resulting acids and subjecting them to successive Arndt-Eistert homologations gives those compounds of formula 5 where n is 1-5. These acids can then be converted to esters of formula I by the procedure outlined above for esterifying the halo-substituted heterocyclic acids.

Amides may be formed by any appropriate amidation means known in the art. In this instance, one way to prepare such compounds is to first make the acid chloride and then treat that compound with ammonium hydroxide. For example, the ester is treated with an alcoholic base such as ethanolic KOH (in approximately a 10% molar excess) at room temperature for about 1/2 hour. The solvent is removed and the residue taken up in an organic solvent such as an ether, treated with a dialkyl formamide and then a 10-fold excess of oxalyl chloride. This is all effected at a reduced temperature between about -10° and +10° C. The last mentioned solution is then stirred at the reduced temperature for 1-4 hours, preferably 2 hours. Removing the solvent leaves a residue which is taken up in an inert solvent such as benzene, cooled to about 0° C and treated with concentrated ammonium hydroxide. The resulting mixture is stirred at a reduced temperature for 1-4 hours. The product is recovered by conventional means.

Alcohols are made by converting the corresponding acids to the acid chloride with thionyl chloride or other means (J. March, Advanced Organic Chemistry, 2nd Edition, McGraw-Hill Book Company), then reducing the acid chloride with sodium borohydride (March, Ibid, page 1124), which gives the corresponding alcohols. Alternatively, esters may be reduced with lithium aluminum hydride at reduced temperatures.

Aldehydes can be prepared from the corresponding primary alcohols using mild oxidizing agents such as pyridinium dichromate in methylene chloride (Corey, E. J., Schmidt, G., Tet. Lett. , 399, 1979 ), or dimethyl sulfoxide/oxalyl chloride in methylene chloride (Omura, K., Swern, D. Tetrahedron, 1978, 34, 1651).

Ketones can be prepared from an appropriate aldehyde by treating the aldehyde with an alkyl Grignard reagent or similar reagent followed by oxidation.

Acetals or ketals can be prepared from the corresponding aldehyde or ketone by the method described

in March, Ibid, p. 810.

Compounds where B is $-CH_3$ are prepared from the corresponding halo-heterocyclic entity preferably where the halogen is I. This haloheterocyclic compound is reacted with the ethynyl zinc chloride entity as described in Reaction Scheme I and more specifically in Example 3. Halo-substituted heterocyclic compounds where B is $-CH_3$ are commercially available or can be prepared by methods in the literature.

The following Examples are set out to illustrate the invention, not to limit its scope.

Example I

1- (2,6,6-Trimethylcyclohex-1-enyl)but-1-ene-3-yne

A solution of 12.17g (120.27 mmol) diisopropylamine in 200 ml dry tetrahydrofuran was cooled to -78°C under argon and treated dropwise via syringe with 75ml of 1.6M (120 mmol) n-butyllithium in hexane. This mixture was stirred at about -78°C for 1 hour and then treated via cannula with a cooled (-78°C) solution of 21.99g (114.35 mmol) β-ionone (1) in 20 ml of dry tetrahydrofuran. This mixture was stirred at about -78°C for 1 hour, treated dropwise with 21.73g (125.93 mmol) of diethyl chlorophosphate and allowed to warm to room temperature over 2 hours. This solution was then transferred by cannula to a solution of lithium diisopropylamide prepared by stirring under argon, a solution of 26.57g (262.57 mmol) diisopropylamine in 150 ml dry tetrahydrofuran and 164 ml of 1.6M (262.4 mmol) n-butyllithium in hexane for 0.5 hour at -78°C. The mixture was allowed to warm to room temperature, stirred for 15 hours, acidified with 250 ml 3N HCl and extracted with pentane. The organic extract was washed with 1N HCl, water, saturated $NaHCO_3$ and saturated NaCl and dried ($MgSO_4$). The product was concentrated and kugelrohr distilled (50°C; 0.1mm) to give the captioned compound as a colorless oil. PMR ($CDCl_3$); δ 1.0 ($2CH_3$, s), 1.45 (2H, m), 1.65 ($CH_3$, s), 1.92 (2H, m) 2.85 (1H, d, J~3Hz), 5.35 (1H, dd, J~16Hz, 3Hz), 6.6 (1H, d, J~ 16 Hz).

Example 2

Ethyl 6-chloronicotinoate

A mixture of 15.75g (0.1 mol) 6-chloronicotinic acid, 6.9g (0.15 mol) ethanol, 22.7g (0.11 mol) dicyclohexylcarbodiimide and 3.7g dimethylaminopyridine, (0.03 mol), in 200 ml of methylene chloride was heated at reflux for 2 hours. The mixture was allowed to cool, solvent removed in vacuo and residue subjected to flash chromatography to give 16.7g of the captioned compound as a low-melting white solid. PMR ($CDCl_3$); δ 1.44 (3H, t, J~6.2Hz), 4.44 (2H, q, J~4.4 Hz), 7.44 (1H, d, J~8.1Hz), 8.27 (1H, dd, J~8.1Hz, 3Hz), 9.02 (1H, d, J~3Hz)

Example 3

Ethyl 6-[4-(2,6,6-trimethylcyclohex-1-enyl)-but-3-ene-1-ynyl]nicotinoate

Reaction vessels used in this procedure were flame dried under vacuum and all operations carried out in an oxygen-free argon or nitrogen atmosphere. To a solution of the Example 1 compound, 620.7 mg (3.5614 mmol), in 4 ml dry tetrahydrofuran at 0°C was added dropwise 2.25 ml of 1.6M (3.6 mmol) n-butyllithinum in hexane. This mixture was stirred at 0°C for 10 minutes at room temperature for 10 minutes and cooled again to 0°C. To this was added, via cannula, a solution of 500 mg (3.6689 mmol) fused zinc chloride in 4ml dry tetrahydrofuran with stirring at 0°C for 1 hour and at room temperature for 10 minutes. A solution of 664mg (3.5774 mmol) ethyl 6-chloronicotinoate in 4 ml of dry tetrahydrofuran was transferred by cannula into a suspension of 430 mg (0.3721 mmol) tetrakis(triphenylphosphine)palladium in 6 ml of dry tetrahydrofuran and stirred for 10 minutes. This mixture was then treated via cannula with the solution of alkynyl zinc and the resultant mixture stirred at room temperature for 60 hours. Water was added (100 ml) and the products extracted with 3×100ml ether. Combined ether extracts were washed with saturated NaCl

solution, dried ($MgSO_4$) and concentrated to give a brown oil. This oil was purified by flash chromatography (silica gel; 10% ethyl acetate in hexanes) followed by high pressure liquid chromatography (Waters 6000; Partisil M-9 10/50; 5% ethyl acetate in hexanes) to give the title compound as a pale yellow oil. PMR ($CDCl_3$); δ 1.06 ($2CH_3$, s), 1.42 (3H, t, J~7Hz), 1.46 (2H, m), 1.61 (2H, m), 1.78 ($CH_3$, s) 2.05 (2H, m), 4.42 (2H, t, J~7Hz), 5.75 (1H, d, J~16.5Hz), 6.89 (1H, d, J~16.5Hz), 7.48 (1H, d, J~7.8Hz), 8.25 (1H, dd, J~7.8, -2Hz), 9.15 (1H, d, J~2Hz).

Proceeding in a similar manner, but substituting for the ethyl 6-chloronicotinoate, the appropriate halo-substituted heterocyclic ester, the following compounds may be prepared:
ethyl 2-[2-(4-(2,6,6-trimethylcyclohex-1-enyl)-but-3-en-1-ynyl)-5-pyridinyl]acetate;
ethyl 3-[2-(4-(2,6,6-trimethylcyclohex-1-enyl)-but-3-en-1-ynyl)-5-pyridinyl]propionate;
ethyl 4-[2-(4-(2,6,6-trimethylcyclohex-1-enyl)-but-3-en-1-ynyl)-5-pyridinyl]butanote; and
ethyl 5-[2-(4-(2,6,6-trimethylcyclohex-1-enyl)-but-3-en-1-ynyl)-5-pyridinyl]pentanoate.

## Example 4

### Ethyl 5-bromo-2-furoate

To a stirred suspension of 8.43g (44.14 mmol) of 5-bromo-2-furoic acid in 100 ml absolute ethanol was added 4 ml of thionyl chloride. This mixture was stirred at reflux for 3 hours and at room temperature for 18 hours. The solvent was removed *in vacuo* the residual oil treated with 100 ml water and extracted with 3 × 75 ml ether. The combined ether extracts were washed with saturated $NaHCO_3$ and saturated NaCl solutions and dried ($MgSO_4$). Solvent was removed *in vacuo* and the residue kugelrohr distilled (60 °C; 0.4mm) to give the captioned compound as a colorless oil. PMR ($CDCl_3$); δ 1.35 (3H, t, J~7Hz), 4.37 (2H, q, J~7Hz), 6.45 (1H, d, J~4Hz), 7.1 (1H, d, J-4Hz).

## Example 5

### Ethyl 5-bromothiophene-2-carboxylate

To 1.092g (5.7157 mmol) of 5-bromothiophene-2-carboxaldehyde was added sequentially, 1.507g (30.75 mmol) sodium cyanide, 60 ml ethanol, 602.5 mg (10.04mmol) of acetic acid and 10.62g (122.16 mmol) of manganese dioxide. This mixture was stirred at room temperature for 24 hours, then filtered through celite and the residue washed several times with ether. The combined filtrates were concentrated, then the residue taken up in water and extracted with 3×75ml ether. Combined ether extracts were washed with saturated $NaHCO_3$, saturated NaCl, dried ($MgSO_4$), concentrated *in vacuo* and kugelrohr distilled (70 °C; 0.1mm) to give the captioned compound as a pale yellow oil. PMR ($CDCl_3$); δ 1.3 (3H, t, J~7Hz), 4.35 (2H, t, J~7Hz), 7.12 (1H, d, J~4Hz), 7.6 (1H, d, J-4Hz).

## Example 6

### Ethyl 5-[4-(2,6,6-trimethyl-cyclohex-1-enyl)-but-3-en-1-ynyl]-2-furoate

### Ethyl 5-[4-(2,6,6-trimethyl-cyclohex-1-enyl)-but-3-en-1-ynyl]thiophene-2-carboxylate

Employing the procedure and conditions described in Example 3, but using instead the ethyl 5-bromo-2-furoate prepared in Example 4 or the ethyl 5-bromo-thiophene-2-carboxylate prepared in Example 5, respectively, the title compounds were prepared. The furoate had the following PMR spectral characteristics: PMR ($CDCl_3$): δ 1.1 (6H, s), 1.43(3H, t, J~7.6Hz), 1.52 (2H, m), 1.65 (2H, m), 1.81 (3H, δ), 2.1(2H, m), 4.42 (2H, q, J~7.6Hz), 5.73 (1H, d, J~16.8Hz), 6.66 (1H, d, J~3.5Hz), 6.83 (1H, d, J~16.8Hz), 7.21 (1H, d, J~3.5Hz). The thiophene-2-carboxylate compound had the following PMR spectral characteristics: PMR-($CDCl_3$):δ 1:08(6H, s), 1.39 (3H, t, J~7.2Hz), 1.50 (2H, m), 1.62 (2H, m), 1.79 (3H, s), 2.08 (2H, m), 4.37 (2H,

q, J~7.5Hz), 5.72 (1H, d, J~16.5Hz), 6.76 (1H, d, J~16.5Hz), 7.14 (1H, d, J~3.9Hz), 7.67 (1H, d, J~3.9Hz).

Proceeding in a similar manner, but substituting for the ethyl thiophene-2-carboxylate and the ethyl 5-bromo-2-furoate, the appropriate heterocyclic ester, the following compounds may be prepared:

ethyl 2-[5-(4-(2,6,6-trimethylcyclohexenyl)-but-3-en-1-ynyl)fur-2-yl]acetate;
ethyl 3-[5-(4-(2,6,6-trimethylcyclohex-1-enyl)-but-3-en-1-ynyl)fur-2-yl]propionate;
ethyl 4-[5-(4-(2,6,6-trimethylcyclohex-1-enyl)-but-3-en-1-ynyl)fur-2-yl]butanote;
ethyl 5-[5-(4-(2,6,6-trimethylcyclohex-1-enyl)-but-3-en-1-ynyl)fur-2-yl]pentanoate;
ethyl 2-[5-(4-(2,6,6-trimethylcyclohex-1-enyl)-but-3-en-1-ynyl)thiophen-2-yl]acetate;
ethyl 3-[5-(4-(2,6,6-trimethylcyclohex-1-enyl)-but-3-en-1-ynyl)thiophen-2-yl]propionate;
ethyl 4-[5-(4-(2,6,6-trimethylcyclohex-1-enyl)-but-3-en-1-ynyl)thiophen-2-yl]butanote; and
ethyl 5-[5-(4-(2,6,6-trimethylcyclohex-1-enyl)-but-3-en-1-ynyl)thiophen-2-yl]pentanoate.

## Example 7

### 6-[4-(2.6.6-Trimethylcyclohex-1-enyl)-but-3-en-1-ynyl]nicotinic acid

Nitrogen gas was bubbled through the solutions used in this experiment immediately before use. To a stirred solution of 53mg (0.1641 mmol) ethyl 6-[4-(2,6,6-trimethylcyclohex-1-enyl)but-3-ene-1-ynyl]-nicotinoate in 200 ml ethanol was added under nitrogen 132ml of a 1.86M (0.2459 mmol) solution of KOH in ethanol and water. After being stirred at room temperature for 3 hours, solvent was removed *in vacuo* and the residue treated with 1 ml water and extracted with 2x1 ml portions of ether. The aqueous layer was then acidified with 50% aqueous acetic acid and extracted with 3x2 ml ether. Combined ether extracts were dried ($MgSO_4$) and concentrated *in vacuo* to give the title product as a pale yellow powder. PMR ($CDCl_3$); δ1.06 (6H, s), 1.48 (2H, m), 1.62 (2H, m), 1.78 (3H, s), 2.05 (2H, m), 5.75 (1H, d, J~16.4Hz), 6.93 (1H, d, J~16.4Hz), 7.55 (1H, d, J~8.1Hz), 8.35 (1H, dd, J~8.1, 2.3Hz), 9.29 (1H, d, J-2.3Hz).

Proceeding in a similar manner, esters prepared according to Example 6 may be converted to the corresponding acid. For example:

5-[4-(2,6,6-trimethyl-cyclohex-1-enyl)-but-3-en-1-ynyl furanoic acid; and
5-[4-(2,6,6-trimethylcyclohex-1-enyl)-but-3-en-1-ynyl]thiophen-2-carboxylic acid.

## Example 8

### 2-[4-(2,6,6-Trimethylcyclohex-1-enyl)-but-3-ene-1-ynyl]-5-hydroxymethylpyridine

A 250 ml 3-necked flask is fitted with a stirrer, a dropping funnel, a nitrogen inlet and a thermometer. In the flask is placed a solution of 379.5 mg (10 mmol) of lithium aluminum hydride in 30 ml of dry diethyl ether. The solution is cooled to -65°C under nitrogen and a solution of 3.2343 g (10 mmol) of ethyl 6-[4-(2,6,6-trimethylcyclohex-1-enyl)-but-3-ene-1-ynyl]-nicotinoate in 15 ml of dry ether is added dropwise at a rate such that the temperature does not exceed -60°C. The mixture is stirred at -30°C for 1 hour and the excess hydride is then destroyed by the addition of 300 mg (3.4 mmol) of ethyl acetate. The reaction mixture is then hydrolyzed by adding 3 ml of saturated ammonium chloride solution and allowing the temperature to rise to room temperature. The mixture is then filtered and the residue washed with ether. The ether layer is then washed with saturated sodium chloride solution, dried ($MgSO_4$) and then concentrated *in vacuo* . The residue is purified by chromatography followed by recrystalliztion to give the title compound.

## Example 9

### 2-[4-(2,6,6-trimethylcyclohex-1-enyl)-but-3-ene-1-ynyl]-5-acetoxymethylpyridine

A solution of 2.81 g (10 mmol) of 2-[4-(2,6,6-trimethylcyclohex-1-enyl)-but-3-ene-1-ynyl]-5-hydroxymethylpyridine, 600 mg (10 mmol) of glacial acetic acid, 2.06 g (10 mmol) of dicyclohexylcarbodiimide

and 460 mg (3.765 mmol) of 4-dimethylaminopyridine in 150 ml methylene chloride is stirred at room temperature for 48 hours. The reaction mixture is then filtered and the residue washed with 50 ml of methylene chloride. The filtrate is then concentrated in vacuo and the residue is purified by chromatography followed by recrystallation to give the title compound.

By the same process, any of the acids or esters prepared in Examples 3 and 6 above may be converted to their corresponding primary alcohol analog.

Example 10

2-[4-(2,6,6-trimethylcyclohex-1-enyl)-but-3-ene-1-ynyl]-pyridine-5-carboxaldehyde

A solution of 1.396 g (11 mmol) of freshly distilled oxalyl chloride in 25 ml of methylene chloride is placed in a 4-necked flask equipped with a stirrer, a thermometer and two pressure-equalizing addition funnels fitted with drying tubes. The solution is cooled to -60 °C and then treated dropwise with a solution of 1.875 g (24 mmol) of dimethyl sulfoxide (distilled from calcium hydride) in 5 ml of methylene chloride over a five minute period. The reaction mixture is then stirred at -60 °C for an additional 10 minutes. A solution of 2.81 g (10 mmol) of 2-[4-(2,6,6-trimethylcyclohex-1-enyl)-but-3-ene-1-ynyl]-5-hydroxymethylpyridine in 10 ml of methylene chloride is then added to the reaction mixture over a period of 5 minutes. The mixture is stirred for a further 15 minutes and is then treated with 5.06 g (50 mmol) of triethylamine. The cooling bath is then removed and the mixture is allowed to warm to room temperature. Thirty ml of water is then added to the mixture and stirring is continued for a further 10 minutes. The organic layer is then separated and the aqueous layer is extracted with 20 ml of methylene chloride. The organic layers are then combined and washed successively with dilute HCl, water and dilute $Na_2CO_3$ solution and then dried ($MgSO_4$). The solution is then filtered and concentrated in vacuo and the residue is purified by chromatography followed by recrystallization to give the title compound.

All alcohols prepared in Example 8 may be oxidized to their corresponding aldehyde by this method.

Example 11

2-[4-(2,6,6-trimethylcyclohex-1-enyl)-but-3-ene-1-ynyl]-5-(1-hydroxypropyl)pyridine

Four ml of a 3M (12 mmol) solution of ethylmagnesium bromide in ether is placed in a 3-necked flask fitted with a mechanical stirrer, a reflux condenser protected by a drying tube and a pressure-equalizing dropping funnel protected by a drying tube. The flask is cooled in an ice-bath and a solution of 2.8 g (10 mmol) of 2-[4-(2,6,6-trimethylcyclohex-1-enyl)-but-3-ene-1-ynyl]-pyridine-5-carboxaldehyde in 10 ml of dry ether is added slowly with vigorous stirring. The cooling bath is then removed and the mixture heated at reflux for 3 hours. The mixture is then cooled in an ice-salt bath and 5 ml of saturated ammonium chloride solution is added. The mixture is stirred for a further 1 hour and then filtered and the residue washed with two 10 ml portions of ether. The ether solution is then separated, dried ($MgSO_4$) and the ether removed in vacuo . The residue is then purified by chromatography followed by recrystallization to give the title compound.

Using the same procedure, but substituting for the pyridine compound noted above, any of the other heteroaromatic aldehydes prepared as per Example 10 can be converted to a secondary alcohol.

Such secondary alcohols may be converted to their corresponding ketone using the same reagents in approximately the same relative amounts of reagent to reactant and essentially the same conditions described in Example 10.

Example 12

2-[4-(2,6,6-trimethylcyclohex-1-enyl)-but-3-ene-1-ynyl]-5-dimethoxymethylpyridine

A round-bottomed flask is fitted with a Dean-Stark apparatus under a reflux condenser protected by a

drying tube. A mixture of 3.35 g (12 mmol) of 2-[4-(2,6,6-trimethylcyclohex-1-enyl)-but-3-ene-1-ynyl]-pyridine-5-carboxaldehyde, 4.80 mg (15 mmol) of anhydrous methanol, 2 mg of p-toluenesulfonic acid monohydrate and 10 ml of anhydrous benzene is placed in the flask and the mixture heated at reflux under nitrogen until close to the theoretical amount of water is collected in the Dean-Stark trap. The reaction mixture is cooled to room temperature and extracted successively with 5 ml of 10% sodium hydroxide solution and two 5 ml portions of water and then dried ($MgSO_4$). The solution is then filtered and the solvent removed in vacuo . The residue is purified by chromatography and then recrystalliztion to give the title compound.

In a similar manner, any aldehyde or ketone of any heteroaromatic containing compound made as per Examples 10 and 11 may be converted to an acetal or a ketal.

## Claims

1. A compound of the formula (I):

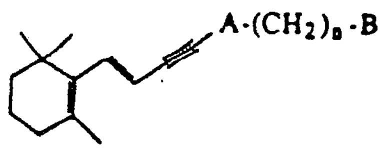

(I)

where
A is pyridyl, furyl, thienyl, pyridazinyl, pyrimidinyl or pyrazinyl;
n is 0-5; and
B is H, -COOH or its esters, amides and pharmaceutically acceptable salts, -CHO and its acetal derivatives, $-CH_2OH$ and its acyl ester derivatives, or $-COR_1$ and its ketal derivatives where $R_1$ is $-(CH_2)_nCH_3$ where n is defined above; or a pharmaceutically acceptable salt, the esters of -COOH and $-CH_2OH$ being derived from the saturated aliphatic alcohols or acids of ten or fewer carbon atoms or the cyclic or saturated aliphatic cyclic alcohols and acids of 5 to 10 carbon atoms, or being phenyl or $C_{1-6}$ alkylphenyl esters; the amides of -COOH being the unsubstituted amides, or the mono- and di-substituted amides derived from the saturated aliphatic radicals of ten or fewer carbon atoms or the cyclic or saturated aliphatic-cyclic radicals of 5 to 10 carbon atoms, or phenyl or $C_{1-6}$ alkylphenyl radicals; the acetals of -CHO and the ketals of $COR_1$ containing a group -CK in place of the carbonyl group, -CK being a group $(OR)_2$ wherein R is $C_{1-6}$ alkyl, or a group $-OR_{1a}O-$ wherein $R_{1a}$ is $C_{2-5}$ alkylene, straight chain or branched.

2. A compound of claim 1 where A is pyridyl.

3. A compound of claim 2 where B is -COOH and its $C_{1-6}$ alkyl esters and pharmaceutically acceptable salts, -CHO, or $-CH_2OH$.

4. A compound according to claim 3 where n is 0, which is
ethyl 6-[4-(2,6,6-trimethylcyclohex-1-enyl)-but-3-en-l-ynyl]nicotinoate, or
6-[4-(2,6,6-trimethylcyclohex-1-enyl)-but-3-en-1-ynyl]nicotinic acid or a pharmaceutically acceptable salt thereof.

5. A compound of claim 1 where A is furyl.

6. A compound of claim 5 wherein B is -COOH and its $C_{1-6}$ alkyl esters, -CHO or $-CH_2OH$, or a pharmaceutically acceptable salt thereof.

7. A compound according to claim 6 where n is 0, which is ethyl 5-[4-(2,6,6-trimethylcyclohex-1-enyl)-but-3-en-1-ynyl]furanoate, or 5-[4-(2,6,6-trimethylcyclohex-1-enyl)-but-3-en-1-ynyl] furanoic acid or a pharmaceutically acceptable salt thereof.

8. A compound of claim 1 where A is thienyl.

9. A compound of claim 8 where B is -COOH and its $C_{1-6}$ alkyl esters, -CHO or $-CH_2OH$ or a pharmaceutically acceptable salt thereof.

10. A compound according to claim 9 where n is 0 which is ethyl 5-[4-(2,6,6-trimethylcyclohex-1-enyl)-but-3-en-1-ynyl]thiophen-2-carboxylate, or 5-[4-(2,6,6-trimethylcyclohex-1-enyl)-but-3-en-1-ynyl]thiophen-2-carboxylicacid.

11. A compound of claim 1 where A is pyridazinyl, pyrimidinyl, or pyrazinyl.

12. A compound of the formula I as defined in any one of claims 1 to 11 for use in medicine.

13. A compound of the formula I as defined in any one of claims 1 to 11 for use in the treatment of psoriasis.

14. A pharmaceutical composition comprising a compound of the formula I as defined in any one of claims 1 to 11 and a pharmaceutically acceptable carrier therefor.

CLAIMS for the following Contracting States: ES, GR

1. A process for making a compound of the formula (I):

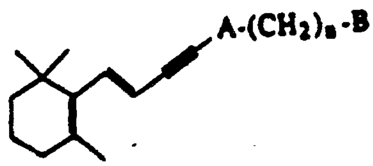

(I)

where
A is pyridyl, furyl, thienyl, pyridazinyl, pyrimidinyl or pyrazinyl;
n is 0-5; and
B is H, -COOH or its esters, and amides, -CHO and its acetal derivatives, $-CH_2OH$ and its acyl ester derivatives, or $-COR_1$ and its ketal derivatives where $R_1$ is $-(CH_2)_n CH_3$ where n is defined above, the esters of -COOH and $-CH_2OH$ being derived from the saturated aliphatic alcohols or acids of ten or fewer carbon atoms or the cyclic or saturated aliphatic cyclic alcohols and acids of 5 to 10 carbon atoms, or being phenyl or $C_{1-6}$ alkylphenyl esters; the amides of -COOH being the unsubstituted amides, or the mono- and di-substituted amides derived from the saturated aliphatic radicals of ten or fewer carbon atoms or the cyclic or saturated aliphatic-cyclic radicals of 5 to 10 carbon atoms, or phenyl or $C_{1-6}$ alkylphenyl radicals; the acetals of - CHO and the ketals of $COR_1$ containing a group -CK in place of the carbonyl group, -CK being a group $(OR)_2$ wherein R is $C_{1-6}$ alkyl, or a group $-OR_{1a}O-$ wherein $R_{1a}$ is $C_{2-5}$ alkylene, straight chain or branched; which process comprises reacting a compound of formula II with a compound of formula III.

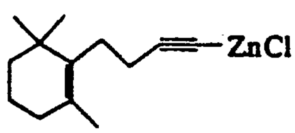

$X-A-(CH_2)_n-B$

II

III

where X is a halogen, preferably I; A is pyridyl, furyl, thienyl, pyridazinyl, pyrimidinyl or pyrazinyl; n is the same as defined above; and B is H, or a protected acid, alcohol, aldehyde or ketone to give a

compound corresponding to formula I.

2. A process according to claim 1 wherein A is pyridyl.

3. A process according to claim 2 wherein B is -COOH and its $C_{1-6}$ alkyl esters and pharmaceutically acceptable salts,
-CHO or $-CH_2OH$.

4. A process according to claim 3 for preparing ethyl 6-[4-(2,6,6-trimethylcyclohex-1-enyl)-but-3-en-1-ynyl]nicotinoate, or 6-[4-(2,6,6-trimethylcyclohex-1-enyl )-but-3-en-1-ynyl]nicotinic acid or a pharmaceutically acceptable salt thereof.

5. A process according to claim 1 wherein A is furyl.

6. A process according to claim 5 wherein B is -COOH and its $C_{1-6}$ alkyl esters, -CHO or $-CH_2OH$, or a pharmaceutically acceptable salt thereof.

7. A process according to claim 6 for preparing ethyl 5-[4-(2,6,6-trimethylcyclohex-1-enyl)-but-3-en-1-ynyl]furanoate, or 5-[4-(2,6,6-trimethylcyclohex-1-enyl)-but-3-en-1-ynyl] furanoic acid or a pharmaceutically acceptable salt thereof.

8. A process according to claim 1 wherein A is thienyl.

9. A process according to claim 8 wherein B is -COOH and its $C_{1-6}$ alkyl esters, -CHO or $-CH_2OH$, or a pharmaceutically acceptable salt thereof.

10. A process according to claim 9 for preparing ethyl 5-[4-(2,6,6-trimethylcyclohex-1-enyl)-but-3-en-1-ynyl]thiophen-2-carboxylate, or 5-[4-(2,6,6-trimethylcyclohex-1-enyl )-but-3-en-1-ynyl]-thiophen-2-carboxylic acid or a pharmaceutically acceptable salt thereof.

11. A process according to claim 1 wherein A is pyridazinyl, pyrimidinyl, or pyrazinyl.

12. A process for making a compound of the formula

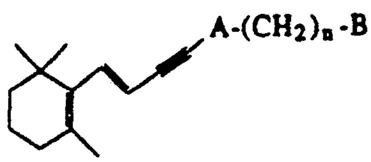

(I)

where
A is pyridyl, furyl, thienyl, pyridazinyl, pyrimidinyl or pyrazinyl;
n is 0-5; and
B is a pharmaceutically acceptable salt of -COOH, which process comprises converting an acid of formula I where B is -COOH or an ester thereof to an acid salt.

13. A process for preparing a pharmaceutical composition comprising bringing a compound of the formula I as defined in any one of claims 1 to 11 into association with a pharmaceutically acceptable carrier.

14. A process for preparing a compound of the formula (I) as defined in claim 1, which process comprises: homologating a compound of the formula

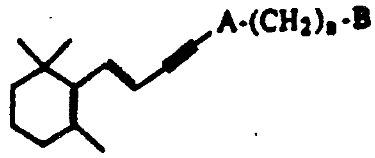

where n is 0-4 and A is a heterocycle as defined in claim 1; or
converting an acid of formula I to an acid or acid salt; or
converting an acid of formula I to an ester; or
converting an acid of formula I to an amide; or
reducing an acid to an alcohol or aldehyde; or
converting an alcohol to an ester; or
oxidizing an alcohol to an aldehyde; or
converting an aldehyde to an acetal; or
converting a ketone to a ketal; wherein the ester, amide, acetal and ketal are as defined in claim 1.

15. A process according to any one of claims 1 to 11 wherein the reaction of the compound of the formula II with the compound of the formula III is conducted in the presence of $Pd(PQ_3)_4$ wherein Q is phenyl.

## Revendications

1. Composé de formule (I)

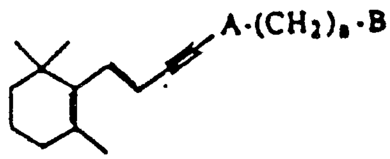

(I)

dans laquelle
A est un reste pyridyle, furyle, thiényle, pyridazinyle, pyrimidinyle ou pyrazinyle;
n est un nombre de 0 à 5; et
B est mis pour H, pour -COOH ou ses esters, amides et sels pharmaceutiquement acceptables, pour -CHO et ses dérivés acétals, pour $-CH_2OH$ et ses dérivés esters acylés ou pour $-COR_1$ et ses dérivés cétals ($R_1$ étant mis pour $-(CH_2)nCH_3$ où n est tel que défini précédemment); ou sel pharmaceutiquement acceptable de ce composé, les esters de -COOH et de $-CH_2OH$ étant dérivés des alcools ou acides aliphatiques saturés à 10 atomes de carbone ou moins ou des alcools et acides cycliques ou alicycliques saturés à 5-10 atomes de carbone ou étant des esters phényliques ou $C_{1-6}$-akylphényliques; les amides de -COOH étant les amides non substitués ou les amides mono- et bisubstitués dérivés des radicaux aliphatiques saturés à 10 atomes de carbone ou moins, des radicaux alicycliques saturés à 5-10 atomes de carbone ou des radicaux phényle ou $C_{1-6}$-alkylphényle; les acétals de -CHO et les cétals de $COR_1$ contenant un groupement -CK à la place du groupement carbonyle, -CK étant un groupement $(OR)_2$ dans lequel R est un reste alkyle en $C_{1-6}$ ou un groupement $-OR_{1a}O$-dans lequel $R_{1a}$ est un radical alkylène en $C_{2-5}$ à chaîne linéaire ou ramifiée.

2. Composé selon la revendication 1, dans lequel A est un reste pyridyle.

3. Composé selon la revendication 2, dans lequel B est mis pour -COOH, ses esters d'alkyle en $C_{1-6}$ et ses sels pharmaceutiquement acceptables, pour -CHO ou pour $CH_2OH$.

4. Composé selon la revendication 3, dans lequel n est mis pour 0 et qui est le 6-[4-(2,6,6-triméthylcyclohex-1-ényl)-but-3-éne-1-ynyl]-nicotinoate d'éthyle, l'acide 6-[4-(2,6,6-triméthylcyclohex-1-ényl)-but-3-éne-1-ynyl]-nicotinique ou un sel pharmaceutiquement acceptable de celui-ci.

5. Composé selon la revendication 1 dans lequel A est un reste furyle.

6. Composé selon la revendication 5, dans lequel B est mis pour -COOH et ses esters d'alkyle en $C_{1-6}$, pour -CHO ou pour $CH_2OH$ ou sel pharmaceutiquement acceptable de celui-ci.

7. Composé selon la revendication 6, dans lequel n est mis pour 0 et qui est le 5-[4-(2,6,6-triméthylcyclohex-1-ényl)-but-3-éne-1-ynyl]-furannoate d'éthyle, l'acide 5-[4-(2,6,6-triméthylcyclohex-1-ényl)-but-3-éne-1-ynyl]-furannoïque ou un sel pharmaceutiquement acceptable de celui-ci.

8. Composé selon la revendication 1 dans lequel A est un reste thiényle.

9. Composé selon la revendication 8, dans lequel B est mis pour -COOH et ses esters d'alkyle en $C_{1-6}$, pour -CHO ou pour $CH_2OH$ ou sel pharmaceutiquement acceptable de celui-ci.

10. Composé selon la revendication 9, dans lequel n est mis pour 0 et qui est le 5-[4-(2,6,6-triméthylcyclohex-1-ényl)but-3-éne-1-ynyl]-thiophène-carboxylate d'éthyle ou l'acide 5-[4-(2,6,6-triméthylcyclohex-1-ényl)-but-3-éne-1-ynyl]-thiophène-2-carboxylique.

11. Composé selon la revendication 1 dans lequel A est un reste pyridazinyle, pyrimidinyle ou pyrazinyle.

12. Composé de formule I tel que défini dans l'une quelconque des revendications 1 à 11, propre à être utilisé en médecine.

13. Composé de formule I tel que défini dans l'une quelconque des revendications 1 à 11, propre à être utilisé dans le traitement du psoriasis.

14. Composition pharmeceutique comprenant un composé de formule I tel que défini dans l'une quelconque des revendications 1 à 11 et un excipient pharmaceutiquement acceptable pour celui-ci.

Revendications pour les Etats Contractants suivants: ES, GR

1. Procédé de préparation d'un composé de formule (I)

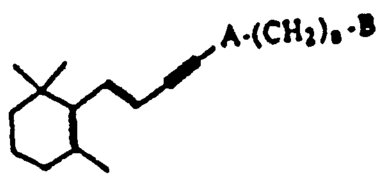

(I)

dans laquelle
A est un reste pyridyle, furyle, thiényle, pyridazinyle, pyrimidinyle ou pyrazinyle;
n est un nombre de 0 à 5; et
B est mis pour H, pour -COOH ou ses esters et amides, pour -CHO et ses dérivés acétals, pour $-CH_2OH$ et ses dérivés esters acylés ou pour $-COR_1$ et ses dérivés cétals ($R_1$ étant mis pour $-(CH_2)-nCH_3$, où n est tel que défini précédemment), les esters de -COOH et de $-CH_2OH$ étant dérivés des alcools ou acides aliphatiques saturés à 10 atomes de carbone ou moins ou des alcools et acides cycliques ou alicycliques saturés à 5-10 atomes de carbone ou étant des esters phényliques ou $C_{1-6}$-alkylphényliques; les amides de -COOH étant les amides non substitués ou les amides mpno- et bisubstitués dérivés de radicaux aliphatiques saturés à 10 atomes de carbone ou moins, des radicaux alicycliques saturés à 5-10 atomes de carbone ou des radicaux phényle ou $C_{1-6}$-alkylphényle; les acétals de -CHO et les cétals de COR, contenant un groupement -CK à la place du groupement carbonyle, -CK étant un groupement $(OR)_2$ dans lequel R est un reste alkyle en $C_{1-6}$ ou un groupement $-OR_1$ O- dans lequel $R_1$. est un radical alkylène en $C_{2-6}$ à chaîne linéaire ou ramifiée, lequel procédé consiste à faire réagir un composé de formule II avec un composé de formule III

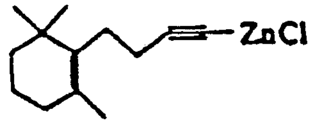

II

$X-A-(CH_2)_n-B$

III

dans lesquelles X est un atome d'halogène, de préférence I; A est un reste pyridyle, furyle, thiényle, pyridazinyle, pyrimidinyle ou pyrazinyle; n est tel que défini précédemment; et B est mis pour H ou pour un acide, alcool, aldéhyde ou cétone protégé, pour obtenir un composé correspondant à la formule I.

**2.** Procédé selon la revendication 1, dans lequel A est un reste pyridyle.

**3.** Procédé selon la revendication 2, dans lequel B est mis pour -COOH, ses esters d'alkyle en $C_{1-6}$ et ses sels pharmaceutiquement acceptables, pour -CHO ou pour $CH_2OH$.

**4.** Procédé selon la revendication 3 pour la préparation de 6-[4-(2,6,6-triméthylcyclohex-1-ényl)-but-3-éne-1-ynyl]-nicotinoate d'éthyle, d'acide 6-[4-(2,6,6-triméthylcyclohex-1-ényl)-but-3-éne-1-ynyl]-nicotinique ou d'un sel pharmaceutiquement acceptable de celui-ci.

**5.** Procédé selon la revendication 1 dans lequel A est un reste furyle.

**6.** Procédé selon la revendication 5, dons lequel B est mis pour -COOH et ses esters d'alkyle en $C_{1-6}$, pour -CHO ou pour $CH_2OH$ ou un sel pharmaceutiquement acceptable de celui-ci.

**7.** Procédé selon la revendication 6 pour la préparation de 5-[4-(2,6,6-triméthylcyclohex-1-ényl)-but-3-éne-l-ynyl]-furanoate d'éthyle, d'acide 5-[4-(2,6,6-triméthylcyclohex-1-ényl)-but-3-ène-1-ynyl]-furannoïque ou d'un sel pharmaceutiquement acceptable de celui-ci.

**8.** Procédé selon la revendication 1 dans lequel A est un reste thiényle.

**9.** Procédé selon la revendication 8, dans lequel B est mis pour -COOH et ses esters d'alkyle en $C_{1-6}$, pour -CHO ou pour $CH_2OH$ ou un sel pharmaceutiquement acceptable de celui-ci.

**10.** Procédé selon la revendication 9 pour la préparation de 5-[4-(2,6,6-triméthylcyclohex-1-ényl)-but-3-éne-1-ynyl]-thiophène-carboxylate d'éthyle, d'acide 5-[ 4- (2,6,6-triméthylcyclohex-1-ényl)-but-3-éne-1-ynyl]-thiophène-2-carboxylique ou d'un sel pharmaceutiquement acceptable de celui-ci.

**11.** Procédé selon la revendication 1 dans lequel A est un reste pyridazinyle, pyrimidinyle ou pyrazinyle.

**12.** Procédé de préparation d'un composé de formule

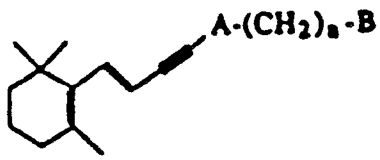

(I)

dans laquelle
A est un reste pyridyle, furyle, thiényle, pyridazinyle, pyrimidinyle ou pyrazinyle;
n est un nombre de 0 à 5; et
B est un sel pharmaceutiquement acceptable de -COOH, lequel procédé consiste à convertir un acide de formule 1 dans laquelle B est mis pour -COOH ou un ester de celui-ci en un sel d'acide.

13. Procédé de préparation d'une composition pharmaceutique, consistant à mettre un composé de formule I tel que défini dans l'une quelconque des revendications 1 à 11 en association avec un excipient pharmaceutiquement acceptable.

14. Procédé de préparation d'un composé de formule (1) tel que défini dans la revendication 1, lequel procédé comprend l'opération consistant
à rendre homologue un composé de formule

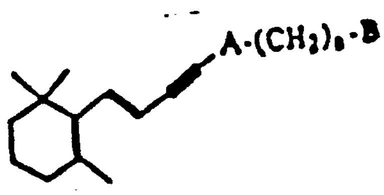

dans laquelle n est un nombre de 0 a 4 et A est un hétérocycle tel que défini dans la revendication 1; ou
à convertir un acide de formule 1 en un acide ou un sel d'acide; ou
à convertir un acide de formule I en un ester; ou
à convertir un acide de formule I en un amide; ou
à réduire un acide en un alcool ou un aldéhyde; ou
à convertir un alcool en un ester; ou
à oxyder un alcool en un aldéhyde; ou
à convertir un aldéhyde en un acétal; ou
ã convertir une cétone an un cétal;
l'ester, amide, acétal et cétal étant tels que définis dans la revendication 1.

15. Procédé selon l'une quelconque des revendications 1 à 11, dans lequel la réaction du composé de formule II avec le composé de formule III est conduite en présence de $Pd(PQ_3)_4$ dans lequel Q est un reste phényle.

**Ansprüche**

1. Verbindung mit der Formel (I):

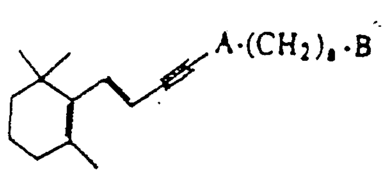

(I)

wobei
A = Pyridyl, Furyl, Thienyl, Pyridazinyl, Pyrimidinyl, oder Pyrazinyl,
n = 0-5 und
B = H, -COOH oder davon die Ester, Amide und pharmazeutisch akzeptablen Salze, -CHO und davon die Acetalderivate, $-CH_2OH$ und davon die Acylesterderivate oder $-COR_1$ und davon die Ketalderivate, wobei $R_1$ = $-(CH_2)n\ CH_3$, wobei n oben definiert ist, oder ein pharmazeutisch akzeptables Salz, wobei die Ester von -COOH und $-CH_2OH$ von den gesättigten aliphatischen Alkoholen oder Säuren mit 10 oder weniger Kohlenstoffatomen oder den cyclischen oder gesättigten aliphatischen cyclischen Alkoholen und Säuren mit 5 bis 10 Kohlenstoffatomen abgeleitet oder Phenyl- oder $C_{1-6}$-Alkylphenylester sind, die Amide von -COOH die unsubstituierten Amide oder die mono- und di-substituierten Amide sind, die von den gesättigten aliphatischen Radikalen von 10 oder weniger Kohlenstoffatomen oder den cyclischen oder gesättigten aliphatisch-cyclischen Radikalen von 5 bis 10 Kohlenstoffatomen oder Phenyl- oder $C_{1-6}$-Alkylphenylradikalen abgeleitet sind, die Acetale von -CHO und die Ketale von $COR_1$ eine -CK-Gruppe anstelle der Carbonylgruppe enthalten und -CK eine $(OR)_2$-Gruppe ist, bei der R ein $C_{1-6}$-

Alkyl oder eine $-OR_{1a}O$-Gruppe ist, bei der $R_{1a}$ ein $C_{2-5}$-Alkylen mit gerader Kette oder verzweigt ist.

2. Verbindung nach Anspruch 1, bei der A = Pyridyl.

3. Verbindung nach Anspruch 2, bei der B = -COOH und davon die $C_{1-6}$-Alkylester und pharmazeutisch akzeptablen Salze, -CHO oder $-CH_2OH$.

4. Verbindung nach Anspruch 3, bei der n = 0, was Ethyl-6-[4-(2,6 ,6-trimethylcyclohex-1-enyl )-but-3-en-1-ynyl]nicotinoat oder 6-[4-(2,6,6-trimethylcyclohex-1-enyl)-but-3-en-1-ynyl]nicotinsäure oder davon ein pharmazeutisch akzeptables Salz ist.

5. Verbindung nach Anspruch 1, bei der A = Furyl.

6. Verbindung nach Anspruch 5, bei der B = -COOH und davon die $C_{1-6}$-Alkylester, -CHO oder $-CH_2OH$ oder davon ein pharmazeutisch akzeptables Salz.

7. Verbindung nach Anspruch 6, bei der n = 0, was Ethyl-5-[4-(2,6,6-trimethylcyclohex-1-enyl )-but-3-en-1-ynyl] furanoat oder 5-[4-(2,6,6-trimethylcyclohex-1-enyl)-but-3-en-1-ynyl]furansäure oder davon ein pharmazeutisch akzeptables Salz ist.

8. Verbindung nach Anspruch 1, bei der A = Thienyl.

9. Verbindung nach Anspruch 8, bei der B = -COOH und davon die $C_{1-6}$-Alkylester, -CHO oder $-CH_2OH$ oder davon ein pharmazeutisch akzeptables Salz.

10. Verbindung nach Anspruch 9, bei der n = 0, was Ethyl-5-[4-(2 ,6,6-trimethylcyclohex-1 -enyl)-but-3-en-1 -ynyl] thiophen-2-carboxylat oder 5-[4-(2,6,6-trimethylcyclohex-1-enyl)-but-3-en-1-ynyl]thiophen-2-carbonsäure ist.

11. Verbindung nach Anspruch 1, bei der A = Pyridazinyl, Pyrimidinyl oder Pyrazinyl.

12. Verbindung nach der Formel I, wie in einem der Ansprüche 1 bis 11 angegeben, zur Verwendung in Medizin.

13. Verbindung nach der Formel I, wie in einem der Ansprüche 1 bis 11 angegeben, zur Verwendung bei der Behandlung von Psoriasis.

14. Pharmazeutische Zusammensetzung, bestehend aus einer Verbindung nach der Formel I, wie in einem der Ansprüche 1 bis 11 angegeben und einem pharmazeutisch akzeptablen Träger für diese.

Patentansprüche für folgende Vertragsstaaten: ES, GR

1. Verfahren zur Herstellung einer Verbindung mit der Formel (I):

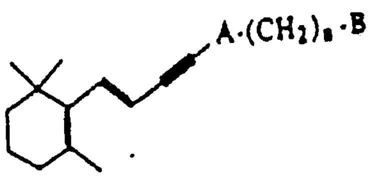

(I)

wobei
A = Pyridyl, Furyl, Thienyl, Pyridazinyl, Pyrimidinyl, oder Pyrazinyl,
n = 0-5 und
B = H, -COOH oder davon die Ester und Amide, -CHO und davon die Acetalderivate, $-CH_2OH$ und davon die Acylesterderivate oder $-COR_1$ und davon die Ketalderivate, wobei $R_1$ = $-(CH_2)nCH_3$, wobei n

oben definiert ist, wobei die Ester von -COOH und -$CH_2OH$ von den gesättigten aliphatischen Alkoholen oder Säuren mit 10 oder weniger Kohlenstoffatomen oder den cyclischen oder gesättigten aliphatischen cyclischen Alkoholen und Säuren mit 5 bis 10 Kohlenstoffatomen abgeleitet oder Phenyl- oder $C_{1-6}$-Alkylphenylester sind, die Amide von -COOH die unsubstituierten Amide oder die mono- und di-substituierten Amide sind, die von den gesättigten aliphatischen Radikalen von 10 oder weniger Kohlenstoffatomen oder den cyclischen oder gesättigten aliphatisch-cyclischen Radikalen von 5 bis 10 Kohlenstoffatomen oder Phenyl- oder $C_{1-6}$-Alkylphenylradikalen abgeleitet sind, die Acetale von -CHO und die Ketale von $COR_1$ eine -CK-Gruppe anstelle der Carbonylgruppe enthalten und -CK eine $(OR)_2$-Gruppe ist, bei der R ein $C_{1-6}$-Alkyl oder eine -$OR_{1a}O$--Gruppe ist, bei der $R_{1a}$ ein $C_{2-5}$-Alkylen mit gerader Kette oder verzweigt ist, welches Verfahren darin besteht, daß eine Verbindung der Formel II mit einer Verbindung der Formel III zur Reaktion gebracht wird,

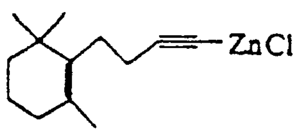

II

$$X-A-(CH_2)_n-B$$

III

wobei X ein Hologen ist, vorzugsweise I, A = Pyridyl, Furyl, Thienyl, Pyridazinyl, Pyrimidinyl oder Pyrazinyl, n gleich der obigen Definition ist und B = H oder eine geschützte Säure, Alkohol, Aldehyd oder Keton, um eine Verbindung entsprechend der Formel I zu ergeben.

**2.** Verfahren nach Anspruch 1, bei dem A = Pyridyl.

**3.** Verfahren nach Anspruch 2, bei dem B = -COOH und davon die $C_{1-6}$-Alkylester und pharmazeutisch akzeptablen Salze, -CHO oder -$CH_2OH$.

**4.** Verfahren nach Anspruch 3, zur Herstellung von Ethyl-6[4-(2,6,6-trimethylcyclohex-1-enyl -but-3-en-1-ynyl]nicotinoat oder 6-[4-(2,6,6-trimethylcyclohex-1--enyl)-but-3-en-1-ynyl]nicotinsäure oder eines pharmazeutisch akzeptablen Salzes davon.

**5.** Verfahren nach Anspruch 1, bei dem A = Furyl.

**6.** Verfahren nach Anspruch 5, bei dem B = -C00H und davon die $C_{1-6}$-Alkylester, -CHO oder -$CH_2OH$ oder davon ein pharmazeutisch akzeptables Salz.

**7.** Verfahren nach Anspruch 6, zur Herstellung von Ethyl-5[4(2,6 ,6-trimethylcyclohex-1 -enyl)-but-3-en-1 -ynyl] furanoat oder 5-[4-(2,6,6-trimethylcyclohex-1-enyl)-but-3-en-1-ynyl]furansäure oder eines pharmazeutisch akzeptablen Salzes davon.

**8.** Verfahren nach Anspruch 1, bei dem A = Thienyl.

**9.** Verfahren nach Anspruch 8, bei dem B = -COOH und davon die $C_{1-6}$-Alkylester, -CHO oder -$CH_2OH$ oder davon ein pharmazeutisch akzeptables Salz.

**10.** Verfahren nach Anspruch 9, zur Herstelung von Ethyl-5-[4-(2,6,6-trimethylcyclohex-1-enyl)-but-3-en-1-ynyl] thiophen-2-carboxylat oder 5-[4-(2,6,6-trimethylcyclohex-1-enyl)-but-3-en-1-ynyl]thiophen-2-carbonsäure oder eines pharmazeutisch akzeptablen Salzes davon.

**11.** Verfahren nach Anspruch 1, bei dem A = Pyridazinyl, Pyrimidinyl oder Pyrazinyl.

**12.** Verfahren zur Herstellung einer Verbindung mit der Formel

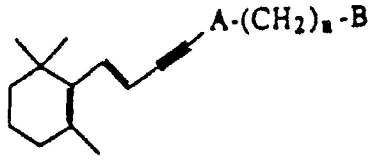

(I)

wobei
A = Pyridyl, Furyl, Thienyl, Pyridazinyl, Pyrimidinyl oder Pyrazinyl,
n = 0-5 und
B ein pharmazeutisch akzeptables Salz von -COOH ist, welches Verfahren darin besteht, daß eine Säure der Formel I, wobei B = -COOH oder davon ein Ester, in ein saures Salz umgewandelt wird.

**13.** Verfahren zur Herstellung einer pharmazeutischen Zusammensetzung, darin bestehend, daß eine Verbindung der Formel I gemäß der Definition in einem der Ansprüche 1 bis 11 in Assoziation mit einem pharmazeutisch akzeptablen Träger gebracht wird.

**14.** Verfahren zur Herstellung einer Verbindung mit der Formel I gemäß Anspruch 1, welches Verfahren darin besteht, daß eine Verbindung der Formel

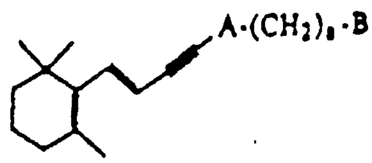

homologiert wird, wobei n = 0-4 und A ein Heterocyclus gemäß Anspruch 1 ist, oder
eine Säure der Formel I in eine Säure oder ein saures Salz umgewandelt wird oder
eine Säure der Formel I in einen Ester umgewandelt wird oder
eine Säure der Formel I in ein Amid umgewandelt wird oder
eine Säure zu einem Alkohol oder Aldehyd reduziert wird oder
ein Alkohol in einen Ester umgewandelt wird oder ein Alkohol zu einem Aldehyd oxidiert wird oder
ein Aldehyd in ein Acetal umgewandelt wird oder
ein Keton in ein Ketal umgewandelt wird, wobei Ester, Amid, Acetal und Ketal so beschaffen sind, wie es im Anspruch 1 angegeben ist.

**15.** Verfahren nach einem der Ansprüche 1 bis 11, bei dem die Reaktion der Verbindung nach der Formel II mit der Verbindung nach der Formel III in Gegenwart von $Pd(PQ_3)_4$ durchgeführt wird, wobei Q = Phenyl.